# EUROPEAN PATENT SPECIFICATION

(11) **EP 2 408 501 B1**
(45) Date of publication and mention of the grant of the patent: **11.05.2016**
(21) Application number: 10713506.3
(22) Date of filing: 05.03.2010
(51) Int. Cl.: A61M 15/00, A61M 16/00, A61M 16/08, A61M 16/14

(54) **CATHETER MOUNT**
KATHETERBEFESTIGUNG
RACCORD DE CATHÉTER

(30) Priority: 06.03.2009 IT BO20090132
(43) Date of publication of application: 25.01.2012
(73) Proprietor: Scarano, Daniele, 84128 Salerno (IT)
(72) Inventor: Scarano, Daniele, 84128 Salerno (IT)
(74) Representative: Mincone, Antimo
(86) International application number: PCT/IB2010/000454
(87) International publication number: WO 2010/100557

(56) References cited:
- WO-A-92/04065
- US-A- 6 014 972
- US-A1- 2002 069 869
- US-A1- 2005 039 746
- US-A1- 2006 254 579
- US-A1- 2008 264 412

## Description

The present invention relates to an improved mount catheter. The mount catheter is a relatively short corrugated tube, which is used, connected to an endotracheal tube, in a oxygenating system, for example of the type called "rebreathing bag". The catheter makes it easier the injection of air, allowing the aspiration.

Furthermore, the mount catheter leaves the possibility to doctors, nurses or rescue teams to be able to move the oxygen systems without moving the endotracheal tube.

In particular, the present invention relates to an improved catheter mount usable for providing nebulized drugs in patients connected to a system of ventilation. In particular, the catheter mount of the invention is advantageously usable in cases of patients who are intubated critically ill, tracheostomised or actively ventilated through a facemask. Currently it is relatively complex to administer a drug to a patient spraying under the above conditions. In fact, it is necessary to suspend the ventilation at least temporarily.

US2005/039746A1 relates to a catheter mount usable for spraying a substance in a ventilation circuit.

The aim of this invention is to allow the nebulization of medications directly within the ventilation circuit which serves the patients, without interruption in the ventilation treatment. Another object of this invention is to increase the efficiency of the drug to the patient.

These and other results are reached in accordance with the invention by adopting the idea of a mount catheter having the features described in claim 1. Other features are described in the dependant claims.

Among the advantages of the present invention are those described below.

The release of the medication sprayed to the patient is achieved without interrupting the ventilation.

There is a better administration of the drug thanks to the catheter which has a larger cross-section, oval shaped; said conformation maintains the drug in the gaseous form (minimizing the deposition of the drug on the walls) and driven by means of the positive airway pressure exerted by the ventilation system (e.g. by means of the rebreathing bag), penetrates better into the airways and increases the therapeutic efficacy.

Advantageously, it is also possible to associate to the ventilation system of an ampoule containing the aerosol drug in liquid form.

Downstream of the oval portion, the catheter includes a section of corrugated pipe; in this way it is possible to rotate of about 45° the oval zone in respect to the joint located downstream, i.e. in proximity of the patient.

In an alternative embodiment of the invention, the ovoid portion consists of two parts which can rotate between themselves; this feature allows the use of a phial/ampoule for spraying aerosol with side port. The possibility of divide this ovoid portion into two parts allows a more effective cleaning and disinfection for the re-use on the same patient.

Each technician who works in this field will better understand these advantages and further advantages and features of the present invention thanks to the following description and to the enclosed drawings as a practical explanation of the present invention which should not be considered in a limitative sense, wherein:
- Fig.1 is a schematic side view of a possible embodiment of a catheter mount in accordance with the invention, in which the catheter is depicted in exploded, with its larger portion divided into two parts;
- Fig.2 is a schematic view of the catheter mount Fig.l, associated with an ampoule containing aerosol;
- Figs.2A, 2B relate to particulars showing a proximal joint represented, respectively, in configuration "free", i.e. without an associated bottle with the drug to be nebulized (2A) and with the bottle in configuration of nebulization (2B);

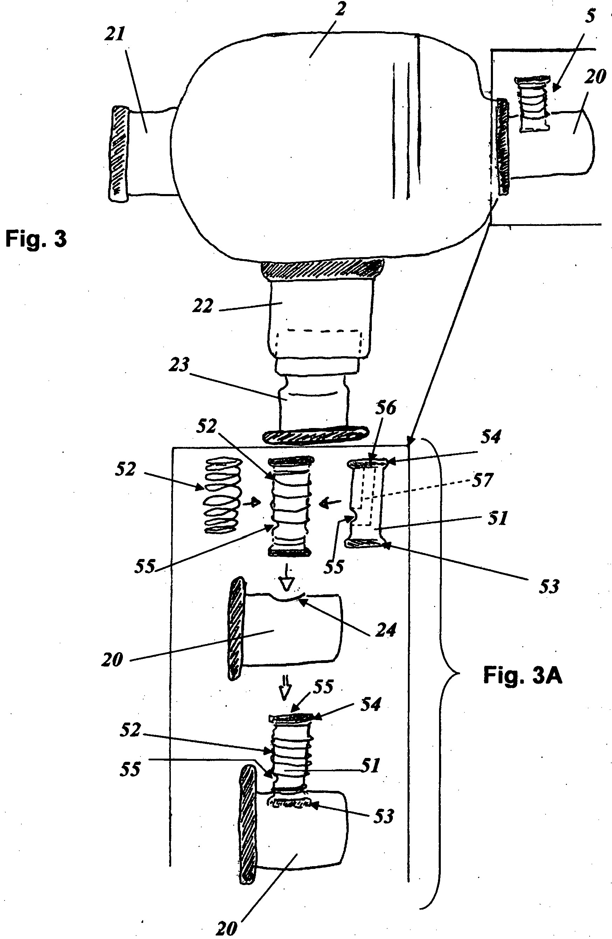

- Fig.3 represents, schematically, the example of Fig.1 in which the portion having the greater section is assembled;
- Fig.3A is relative to some details of the proximal joint of the catheter mount;
- Figs.4, 4A, 4B illustrate the catheter mount of the invention in different configurations of use which can be obtained by relative rotation of the two parties that form the largest portion of the catheter;
- Figs. 5, 6 show schematically some possible uses of the mount catheter of the invention; and
- Fig 7 shows an improved mount catheter according to the invention.

With reference to the examples of the drawings, a mount catheter (1) in accordance with the invention can be used for spraying a substance in a ventilation circuit comprising means (4) acts to send a ventilation flow and means for the connection (3) to a patient.

In the examples, which are not exhaustive of other possible embodiments, the means (4) for sending a ventilation stream consist of a socalled rebreathing bag or "Ambu ball".

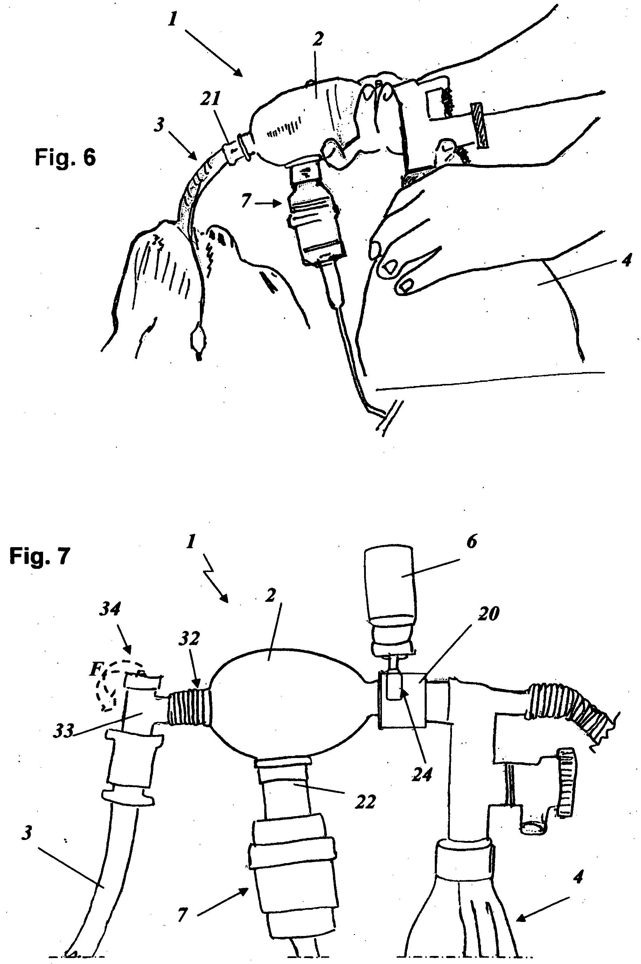

Also the means for the connection (3) to a patient can be of different type: tight face mask, tracheostomy tube, oro-tracheal tube; these means are connected to the beak-junction which is placed at the proximal end of the catheter mount. (as in Fig 7).

The catheter mount (1) is provided with a portion having a larger section (2) interposed between the means (4) for sending the ventilation flow and the means (3) for the connection to a patient; said portion having a larger section (2) is provided with a proximal joint (20) and a distal joint (21; 34); said joints are apt to connect, respectively, downstream to said means of connection (3) to a patient and upstream to said means (4) for sending the ventilation flow. On the proximal joint (20) is disposed an opening or port (24) usable for attaching connecting resolvable means (5) for the connection to a container (6) of substances to be nebulized.

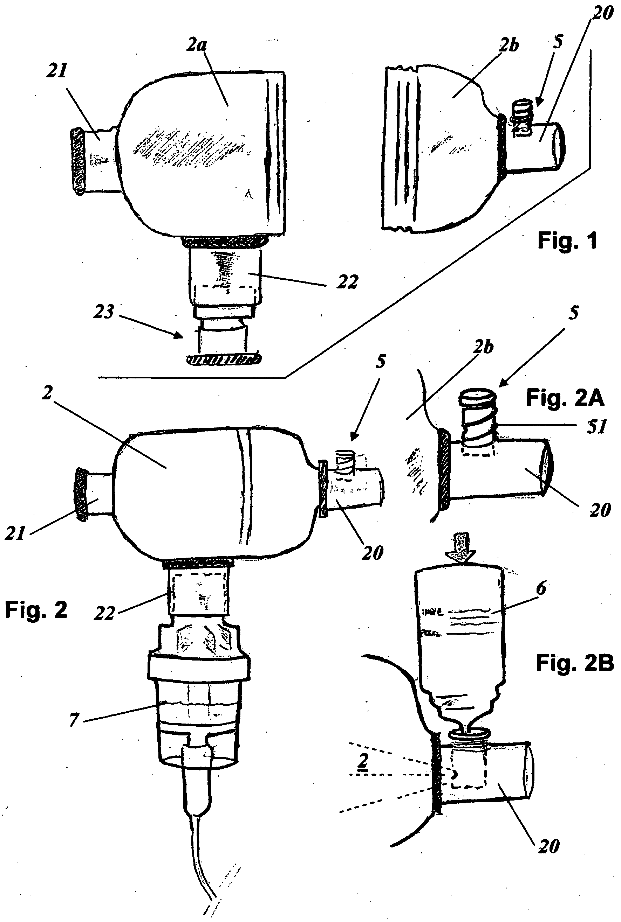

More specifically, with reference to Figs. 1, 2, 4 and 5, the portion (2) has a substantially oval shape and is formed of two parts: a proximal part (2b) and a distal one (2a) coupled in a stable but reversible way, for example with an interlocking joint.

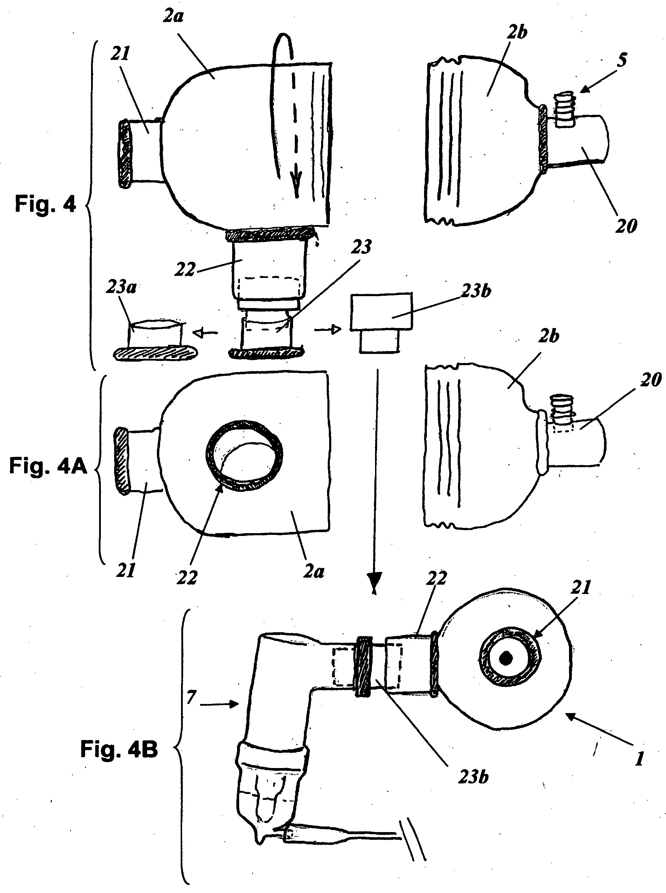

It is possible to realize the connection between the two parts (2a) and (2b) in such a manner that it is allowed the rotation of the distal portion (2a) in respect to the proximal portion (2b). This feature further enhances the functionality of the catheter mount to use a cruet/ampoule of aerosol spray (7) with side port, as shown in the drawings of Figs.4-4B.

Moreover, the possibility of separating the oval portion (2) into two parts allows a more thorough cleaning and disinfection for reuse.

The proximal portion (2b) is provided with said proximal joint (20), which consists of a hollow cylinder (20) whose shape enables the tight connection with the means of ventilation (the rebreathing ball 4 of the examples). The hollow cylindrical body (20) communicates with the downstream ovoid portion ovoid (2) and, upstream, with the rebreathing ball (4).

Said opening or port (24) is disposed radially on the joint (20), that is, on its cylindrical wall, particularly in an area of said joint (20) which, in use, is disposed on the top.

The door (24) of the junction (20) is crossed by an introducer consisting of tubular body (51) provided with ends having a greater section with edges (53, 54). In practice, the edges (53) and (54) of the tubular body (51) are sized so they do not pass through the opening (24).

The tubular body (51) is provided with an internal channel (57) having an inlet (56) disposed at the top, that is, on its upper base (that is bounded by the edge 54), and an output (55) radially disposed in respect to the tubular body (51), in an area intended to be inside the joint (20) when the tubular body (51) is inserted within the same joint (20). In particular, the output (55) is facing downstream, that is to direct the flow towards to the ovoid portion (2).

The catheter mount can comprise means for preventing the rotation of the tubular body (51) when the same is cylindrical in shape; for example, it can comprise a vertical guide groove which, by preventing the rotation of the body (51) around its longitudinal axis, determines the positioning of the output (55) in direction of the oval portion (2). Alternatively, the tubular body (51) may have a polygonal section, without the necessity of a guide groove. On the body (51) is fit a spring (52) whose ends are batting, respectively, against the upper surface of the joint (20) and against the lower edge (54) of the body (51). The compression due to the pre-loading of the spring (52) pushes the tubular body (51) to the outside in respect to the junction (20) in which it is partially inserted. In practice, the tubular body (51) is held normally "extracted", pushed to the outside and with its lower edge (53) batting against the inside face of the junction (20), around the opening (24).

The inlet (56) of the tubular body (51) is shaped so as to be coupled with the supplying nozzle of the container of the spray drugs.

For administering nebulized medications, it is sufficient to simply juxtapose the dispensing nozzle of the container at the upper hole (56), exerting a pressure on the same bottle, as indicated by the arrow in Fig.2B. The force exerted on the bottle will move the tubular body (51) inside the cavity of the junction (20), loading the spring (52); the medicine sprays out from the container (6) and passing through the channel (57) and the exit (55) is received nebulized within the oval portion (2).

Once the pressure on the bottle (6) ceases, the tubular body (51), due to the elastic recoil of the spring (52), is reported out of the cavity (20).

Simultaneously, the balloon (4) is squeezed, practicing so to the patients a forced ventilation which pushes actively (with positive pressure) through the respiratory tree the mixture 02-medication.

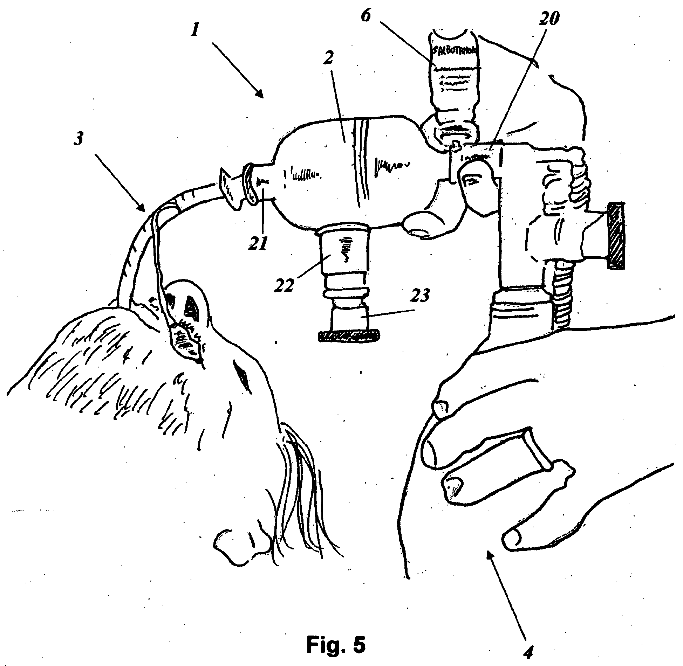

The distal portion (2a) of the oval portion (2) has two holes: a first hole disposed centrally (axially), in correspondence of the distal joint (21), another hole disposed on the side (radially in respect to the longitudinal development of the egg-shaped portion 2), in correspondence of a port or additional joint (22) constituted of a hollow cylinder. The distal junction (21; 34) may be cylindrical (21) and can connect directly to the oro-tracheal tube or tracheal cannula (Figs. 5, 6) or it can be beak-shaped (34), in this latter case, there is a corrugated part (32) which is the continuation of oval portion and which has a joint (33) for the device directed to the patient in position orthogonal in respect to the corrugated portion (Fig. 7).

The additional joint (22), if not used as a gateway for the continuous delivery of nebulized drugs, is maintained sealed with a adapter cap (23). The cap is composed of two parts: a cap (23a) and an adapter (23b).

For allowing the continuous delivery of nebulized drugs, once removed the cap (23), it is possible to connect to the oval portion (2) an ampoule (7) for aerosol having a vertical connection (such as schematically illustrated in Fig.2) or side connection (see Figs. 4B). In the latter case it is necessary first to rotate the camera about 45° and then use the upper part (23b) of the cap (23) which acts as an adapter. This feature allows to increase the possibilities of connection between different diameter pipes. When the oval portion consists of a single body, the directional rotation of the oval enlarged portion allows the use of the additional joint (22) by rotating the beak joint (see arrow F of Figure 7).

The presence of this additional access to the chamber defined by the enlarged portion thus allows many advantages when considering the benefits from the use of drugs directly involved in the respiratory tracts of acute and chronic pulmonary diseases, both primary and secondary.

In conclusion, it is possible to affirm that by means of the catheter mount of the invention the drug nebulizers, that otherwise could not be used, can be used (with the rebreathing ball or Ambu ball) for critical uncooperating patients (i.e., intubated or active ventilated patients) or for cooperating patients (tracheostomised), said patients having conditions which are progressively deteriorating.

It is possible the connection of the ampoule containing the drug in liquid form to a machine for aerosol or a source of oxygen at high flow.

The catheter mount (1) shown in Figure 7, is provided with a greater portion of ovoid-section (2) which downstream narrows into a smaller cross section portion (32) for connection with a beak joint (33) to which is associated the tube (3) attached to the patient. The lower section (32) and the beak joint (33) are the distal joint (34) of the greater cross-section portion (2) of the mount (1). In particular, the portion of lower section (32) is formed by a corrugated tube that allows rotation of about 45° of the larger section portion.

Furthermore, the oval portion (2) are provided, as in the examples described above, with a port (24) disposed at the proximal junction (20) that connects the re-breathing ball (4), and one additional port (22) for the association of another device, such as an ampoule (7) containing the drug in liquid form (aerosol).

The additional joint (22) is advantageously placed in correspondence of the central portion of the oval portion (2), i.e. at the greatest cross-section; this particular feature increases the effectiveness of drug treatment because it enables optimal distribution of the drug in the flow of air directed to the patient.

In conclusion, the present invention relates, in particular, to an improved catheter mount usable for administering nebulizable drugs to patients connected to a system of ventilation. The use of the catheter mount of the invention makes it extremely simplified the administration of these drugs in intubated critically ill patients, tracheostomised or actively ventilated through facemask. In other words, the administration of these drugs, which would be considerably complicated with known type devices is extremely easy even in emergencies, in which small changes in the treatment and / or temporary changes in ventilation can cause severe damage to the patient.

The execution details may equally vary as regards shape, size, disposition of elements, kind of materials used, within the solution idea that has been adopted and within the limits of the protection offered by the present patent.

## Claims

1. A catheter mount usable for spraying a substance in a ventilation circuit comprising means for sending a ventilation flow (4) and means for the connection to a patient (3); said catheter mount (1) wherein:
- the catheter mount comprises a larger cross-section portion (2) interposed between said means for sending the ventilation flow (4) and said means for the connection to a patient (3),
- said larger cross-section portion (2) being provided with a proximal joint (20) for the upstream connection to said means for sending a ventilation flow (4) and a distal joint (21, 34) for the downstream connection to said means for the connection to a patient (3) respectively,
- said proximal joint (20) being provided with a port (24) suitable for fixing a connection means (5) for reversibly connecting a container (6) of substances apt to be sprayed,
- the catheter mount further comprising said connection means (5),
- said connecting means (5) comprises a tubular introducer body (51),
- said tubular body (51) can pass through the port (24) and is provided with an internal channel (57) provided with an input (56) matchable with said container (6) and an output (55) disposed in an area which is inside the proximal joint (20) when the tubular body (51) is inserted within the proximal joint (20);
- said tubular body (51) is further provided with ends having edges (53, 54) sized so they do not pass through said port (24),
- the connection means (5) further comprising a spring (52) fitted on said tubular body (51).

2. A catheter mount according to claim 1, **characterized in that** downstream of said larger cross-section portion (2), the distal joint comprises a portion of corrugated tube (32).

3. A catheter mount according to one of the preceding claims, **characterized in that** downstream of said larger cross-section portion (2), the distal joint comprises a portion of corrugated tube (32) and a beak joint (33).

4. A catheter mount according to claim 1, **characterized in that** there are means for positioning said output (55) for keeping it facing downstream, for directing the relevant flow towards the larger cross-section portion (2).

5. A catheter mount according to claim 1, **characterized in that** said larger cross-section portion (2) is formed by a proximal part (2b) and a distal part (2a) coupled each other in a stable and reversible way.

6. A catheter mount according to claim 5, **characterized in that** said larger cross-section portion (2) is formed by a proximal part (2b) and a distal part (2a) coupled each other in a stable and reversible way; one of said parts being provided with an additional joint (22), said two parts (2a, 2b) of the chamber being reciprocally connected in such a manner that it is allowed the rotation of the distal portion (2a) in respect to the proximal portion (2b).

7. A catheter mount according to claim 1, **characterized in that** said larger cross-section portion (2) is of ovoid shape.

## Patentansprüche

1. Tubusverlängerung, die für das Zerstäuben von Substanzen in einem Beatmungskreislauf verwendet werden kann, das Mittel (4) umfasst, die fähig, sind, den Beatmungsfluss mittels eines Verbindungsstückes (3) einem Patienten zuzuführen; besagte Tubusverlängerung (1), in dem:
- die Tubusverlängerung einen Teil mit einem größeren Querschnitt (2) umfasst, das sich zwischen den besagten Mitteln (4) für die Zuführung des Beatmungsflusses zu einem Patienten und den besagten Verbindungsstücken (3) befindet,
- besagtes Teil mit größeren Durchschnitt (2) verfügt über einen proximalen Anschluss (20) für die obere Verbindung mit den besagten Mitteln (4), die fähig sind, den Beatmungsfluss zuzuführen und über einen distalen Anschluss (21; 34) für die untere Verbindung mit den besagten Verbindungsmitteln (3) zu einem entsprechendem Patienten,
- besagter proximaler Anschluss (20) verfügt über eine Pforte (24), die für die Anbringung von lösbaren Anschlussmitteln (5) an einen Behälter (6) für zerstäubbare Substanzen verwendbar ist,
- die Tubusverlängerung umfasst außerdem sieben Verbindungsstücke (5),
- die besagten Verbindungsmittel (5) umfassen einen Körper für das Einführen (51),
- besagter schlauchartiger Körper (51) kann über dieselbe Pforte (24) passieren und verfügt über einen internen Kanal (57), der über einen Zugang (56), der mit dem besagten Behälter (6) verbunden werden kann, und über einen Ausgang (55), der sich in einer im Inneren des proximalen Anschluss (29) liegenden Zone befindet, wenn der schlauchförmige Körper (51) ins Innere des proximalen Anschlusses (20) eingeführt ist, verfügt.
- besagter schlauchartiger Körper (51) verfügt außerdem über ein Ende mit Rändern (53, 54), die so dimensioniert sind, dass sie nicht in die besagte Öffnung (24) passen,
- die Verbindungsmittel (5) umfassen außerdem eine über den besagten schlauchförmigen Körper (32) gestülpte Feder (52).

2. Tubusverlängerung nach Anspruch 1, charakterisiert durch die Tatsache, dass der distale Anschluss unterhalb des besagten Teils mit einem größeren Durchschnitt (2) einen Teil aus einem Wellrohr (32) umfasst.

3. Tubusverlängerung nach einer der vorherigen Ansprüche, charakterisiert durch die Tatsache, dass unterhalb des besagten Teils mit einem größeren Durchschnitt (2) der distale Anschluss (34) ein Teil aus Wellrohr (32) und einen Schnabelanschluss (33) umfasst.

4. Tubusverlängerung nach Anspruch 1, charakterisiert durch die Tatsache, dass Mittel für die Positionierung für den besagten Ausgang (55) vorgesehen sind, die geeignet sind, diesen nach unten zu halten, sodass der betroffene abgehende Fluss in den Teil mit dem größeren Durchschnitt geleitet wird (2).

5. Tubusverlängerung nach Anspruch 1, charakterisiert durch die Tatsache, dass der besagte Teil mit größerem Durchschnitt (2) aus einem proximalen (2b) und einem distalen (2a) Teil besteht, die stabil und lösbar sind.

6. Tubusverlängerung nach Anspruch 5, charakterisiert durch die Tatsache, dass die besagte Kammer (2) aus einem proximalen Teil (2b) und einem distalen (2°) Teil besteht; eine dieser Kammern verfügt über einen zusätzlichen Anschluss (22), die besagten zwei Teile (2a, 2b) der Kammer sind zueinander in verschiedenen Winkeln positionierbar.

7. Tubusverlängerung nach Anspruch 1, charakterisiert durch die Tatsache, dass besagter Teil mit größerem Durchschnitt (2) eine eiförmige Form hat.

## Revendications

1. Un raccord de cathéter utilisable pour la pulvérisation d'une substance dans un circuit de ventilation comprenant des moyens (4) pour l'envoi d'un flux de ventilation et des moyens de connexion (3) à un patient; ledit raccord de cathéter (1) dans laquelle:
- raccord de cathéter comprend une portion de section transversale plus grande (2) interposés entre lesdits moyens (4) pour envoyer le flux de ventilation et lesdits moyens (3) pour la connexion à un patient,
- ladite portion de section transversale plus grande (2) étant pourvue d'un joint proximal (20) pour le raccordement en amont auxdits moyens (4) pour l'envoi d'un flux de ventilation et un joint distal (21; 34) pour la connexion en aval auxdits moyens pour la connexion (3) à un patient, respectivement,
- ledit joint proximal (20) étant pourvue d'une porte (24) adaptée pour la fixation des moyens de connexion (5) pour connecter de manière réversible un récipient (6) de substances aptes à être pulvérisée,
- le raccord de cathéter comprenant en outre lesdits moyens de connexion (5),
- lesdits moyens de connexion (5) comprennent un corps introducteur (51),
- ledit corps tubulaire (51) peut passer à travers le même port (24) et est pourvu d'un canal interne (57) muni d'une entrée (56) rattachable audit récipient (6) et une sortie (55) disposé dans une zone qui est à l'intérieur du joint proximale (20) lorsque le corps tubulaire (51) est inséré dans le joint proximale (20);
- ledit corps tubulaire (51) est en outre muni d'extrémités ayant des bords (53, 54) dimensionnés de telle sorte qu'ils ne passent pas à travers ladite porte (24),
- les moyens de connexion (5) comprenant en outre un ressort (52) monté sur ledit corps tubulaire (51).

2. Un raccord de cathéter selon la revendication 1, **caractérisé en ce qu'**en aval de ladite portion de section transversale plus grande (2), le joint distal comprend une portion de tube ondulé (32).

3. Un raccord de cathéter selon l'une quelconque des revendications précédentes, **caractérisé en ce qu'**en aval de ladite portion de section transversale plus grande (2), le joint distal comprend une portion de tube ondulé (32) et un joint à bec (33).

4. Un raccord de cathéter selon la revendication 1, **caractérisé en ce qu'**il y a des moyens pour positionner ladite sortie (55) pour le maintenir orienté vers l'aval, pour diriger le flux concerné vers ladite portion de section transversale plus grande (2).

5. Un raccord de cathéter selon la revendication 1, **caractérisé en ce que** ladite portion de section transversale plus grande (2) est formée par une partie proximale (2b) et une partie distale (2a) couplés les uns des autres d'une manière stable et réversible.

6. Un raccord de cathéter selon la revendication 5, **caractérisé en ce que** ladite portion de section transversale plus grande (2) est formée par une partie proximale (2b) et une partie distale (2a) couplés les uns des autres d'une manière stable et réversible; l'une desdites parties étant munie d'un joint supplémentaire (22), lesdites deux parties (2a, 2b) de la chambre étant reliée mutuellement de telle sorte qu'il soit permis à la rotation de la partie distale (2a) par rapport à l'extrémité proximale partie (2b).

7. Un raccord de cathéter selon la revendication 1, caractérisé en ce ladite portion de section transversale plus grande (2) est de forme ovoïde.
